# EUROPÄISCHE PATENTSCHRIFT

(11) **EP 1 100 509 B1**
(45) Veröffentlichungstag und Bekanntmachung des Hinweises auf die Patenterteilung: **06.04.2005**
(21) Anmeldenummer: 99936276.7
(22) Anmeldetag: 13.05.1999
(51) Int. Cl.: A61K 31/565, A61P 15/12

(54) **VERWENDUNG VON BIOGENEN ESTROGENSULFAMATEN ZUR HORMONSUBSTITUTIONSTHERAPIE**
USE OF BIOGENIC ESTROGEN SULFAMATES FOR HORMONE REPLACEMENT THERAPY
UTILISATION DE SULFAMATES D'OESTROGENES BIOGENES POUR L'HORMONOTHERAPIE SUBSTITUTIVE

(30) Priorität: 28.07.1998 DE 19834931
(43) Veröffentlichungstag der Anmeldung: 23.05.2001
(73) Patentinhaber: Schering Aktiengesellschaft, 13353 Berlin (DE)
(72) Erfinder: ELGER, Walter, D-14195 Berlin (DE); LÄHTEENMÄKI, Pekka, FIN-20900 Turku (FI); LEHTINEN, Matti, FIN-20760 Piispanristi (FI); REDDERSEN, Gudrun, D-07749 Jena (DE); ZIMMERMANN, Holger, D-98693 Ilmenau-Roda (DE); OETTEL, Michael, D-07743 Jena (DE); SCHWARZ, Sigfrid, D-07743 Jena (DE)
(74) Vertreter: Ziebig, Marlene, Dr. Dipl.-Chem.
(86) Internationale Anmeldenummer: PCT/DE1999/001496
(87) Internationale Veröffentlichungsnummer: WO 2000/006175

(56) Entgegenhaltungen:
- WO-A-95/01161
- WO-A-97/33589
- US-A- 5 633 242
- ELGER, W. ET AL: "Novel oestrogen sulfamates: a new approach to oral hormone therapy" EXPERT OPINION INVEST. DRUGS, Bd. 7, Nr. 4, 1998, Seiten 575-589, XP002121926
- ELGER, W. ET AL: "Sulfamates of various estrogens are prodrugs with increased systemic and reduced hepatic estrogenicity at oral application." JOURNAL OF STEROID BIOCHEMISTRY AND MOLECULAR BIOLOGY, Bd. 55, Nr. 3-4, 1995, Seiten 395-403, XP002026379

## Beschreibung

Die Erfindung betrifft die Verwendung von biogenen Estrogensulfamaten zur oralen, diskontinuierlichen Applikation zur Hormonsubstitutionstherapie (HRT).

Estrogene werden überwiegend von Graafschen Follikeln und dem Gelbkörpern im Ovar gebildet. Daneben sind viele Organe und Gewebe in der Lage, Estrogene zu generieren, zum Beispiel aus Androstendion und Dehydroepiandrosteron, die von der Nebenniere des Menschen in beträchtlicher Menge sezerniert werden. An der entsprechenden Umwandlung sind unter Umständen mehrere Enzyme und am Ende der Kette schließlich die Aromatase beteiligt. Ein anderer Weg der Entstehung von Estrogenen im Gewebe ist die hydrolytische Spaltung von Konjugaten der natürlichen Estrogene, in erster Linie der des Estronsulfates. Es ist davon auszugehen, daß im Gewebe entstehende Estrogene lokal eine wichtige Rolle in physiologischen und pathologischen Prozessen spielen. Allerdings sind sie nicht in der Lage, das Estrogendefizit im Gesamtorganismus zu verhindern, das mit dem Erlöschen der Ovarialfunktion um das 50. Lebensjahr eintritt.

Estrogene spielen in der hormonalen Kontrazeption und in der klimakterischen Hormon-Replacement-Therapie (HRT) sowie bei der Behandlung gynäkologischer (z. B. Mammacarcinom) und andrologischer (z. B. Prostatacarcinom) Krankheitsbilder eine wesentliche Rolle. Im Falle der Kontrazeption werden Estrogene einmal dazu benötigt, um Follikelreifung und Ovulation sicher zu unterdrücken, andererseits substituieren sie dann die weitgehend unterdrückte endogene, ovarielle Sekretion von Estradiol. Diese Substitution ist wesentlich für die Erhaltung eines artifiziellen Menstruationszyklus und anderer Funktionen der Sexualorgane, die mit einem Gestagen allein nicht befriedigend gelingt. Daneben haben endogene und exogene Estrogene wichtige zentralnervöse und metabolische Funktionen im weiblichen Organismus. Normale Estrogenspiegel tragen zum Wohlbefinden entscheidend bei. Ihre Anwesenheit wirkt dem Entstehen von Herz-Kreislauf-Erkrankungen über verschiedene Mechanismen entgegen: Erzeugung von "günstigen" Lipoproteinmustern im Blut, Hemmung der Lipideinlagerung in der Gefäßwand, Senkung des Blutdrucks durch günstige Beeinflussung des Gefäßtonus, Reduktion des Perfusionswiderstandes in wichtigen Gefäßgebieten, Dämpfung kontraktiler Reize am Gefäßmuskel. Unter der Wirkung von Estrogenen setzen die Gefäßinnenwände Faktoren frei, die der Entstehung von Blutgerinnseln entgegen wirken. Estrogene sind bei der Frau zur Erhaltung der Knochenstruktur unerläßlich. Ihr Verlust kann die Entwicklung eines Knochenabbaus (Osteoporose) bewirken. Die letztgenannten "zentralnervösen" und "metabolischen" Effekte der Estrogene sind wesentlicher Gesichtspunkt der HRT.

Bei allen positiven Aspekten der Estrogentherapie gibt es ungelöste Probleme, welche die therapeutische Anwendung von Estrogenen einschränken oder unerwünschte Wirkungen beinhalten, diese werden im Hinblick auf den Gegenstand der Erfindung in den folgenden Kapiteln erörtert.

Natürliche Estrogene (Estradiol, Estron, Estronsulfat, Ester von Estradiol, Estriol) werden bei oraler Anwendung nur zum geringsten Teil bioverfügbar. Dieser Anteil ist individuell so variabel, daß generelle Dosisempfehlungen nicht möglich sind. Die Estrogendosis in der HRT muß sehr oft individuell angepaßt werden. Problematisch ist auch die rasche Eliminierung der Substanzen aus dem Blut. Selbst bei täglicher Einnahme eines oralen Präparates werden der Wirkstoff und seine relevanten Metabolite zwischen zwei Einnahmen so weitgehend eliminiert, daß nicht davon ausgegangen werden kann, daß dies nicht zu einer Störung der estrogenen Wirkung führt. In Untersuchungen von Kuhnz et al. (Kuhnz W, Gansau C, Mahler M: "Pharmacokinetics of Estradiol, Free and Total Estrone, in Young Women Following Single Intravenous and Oral Administration of 17 β-Estradiol", Arzneim. -Forsch./Drug Res. **43 (2),** 9, 966 - 973 (1993)) wurde gefunden, daß die Estradiol- und Estronwerte 24 Stunden nach Applikation unterschiedlicher Dosen von Estradiol (2, 4 und 8 mg als Einmalapplikation) auf weniger als 50% der maximalen Spiegel gefallen waren. Diese Beobachtung zeigt, daß Dosiserhöhung keineswegs in der Lage ist, das Problem der starken Schwankungen der Hormonspiegel im 24-Stundenrhythmus bei täglicher Einnahme zu beheben. Die Relevanz dieser Annahme kann auch durch andere Beobachtungen gestützt werden. Estriol war bei postmenopausalen Frauen auch bei sehr hohen oralen Dosierungen nicht osteoprotektiv wirksam (Lindsay R, Hart D M, Maclean A, Garwood J, Clark A C, Kraszewski A: "Bone loss during estriol therapy in postmenopausal women" Maturitas Jun **1 (4)**, 279 - 285 (1979)). Es hat bei der Frau eine besonders kurze Halbwertszeit von circa 1,5-5,3 Stunden (Heithecker R, Aedo A R, Landgren B M, Cekan S Z: "Plasma Estriol Levels after Intramuscular Injection of Estriol and Two of Its Esters" Horm. Res. **35,** 234 - 238 (1991)). Es wurde nachgewiesen, daß dieses Estrogen nach Ovarektomie im Knochen protektiv wirkt, wenn gleichmäßige Wirstoffspiegel im Blut aufrecht erhalten werden (Elger W, Schneider B, Oettel M, Ernst M, Hübler D, Dittgen M: "Verwendung von Oestriol zur Behandlung von klimakterischer Osteoporose" Patent DE-A 42 09 295).

In den letzten Jahren wurden transdermale Therapieverfahren entwickelt. Diese reduzieren die Fluktuation der Estrogenspiegel im Blut, können diese aber nicht ganz vermeiden. Der wesentliche Nachteil dieser Applikationstechnologie liegt vermutlich in der komplizierteren Anwendung im Vergleich zur einfachen oralen Applikation. Orale Präparate beherrschen trotz ihrer diskutierten Nachteile den HRT-Markt nach wie vor. Transdermale Anwendungsformen werden zudem im Mittel von ihren Anwenderinnen früher aufgegeben als das für orale Präparate der Fall ist.

Diese werden von der medizinischen Wissenschaft mit Hinweis auf ihre metabolischen Effekte für das Anwendungsgebiet HRT abgelehnt. Das Wichtigste synthetisch abgewandelte estrogene Steroid ist das Ethinylestradiol (EE). Dieses Estrogen ist beherrschend in der oralen hormonalen Kontrazeption. Neben EE wird in wenigen Produkten das Mestranol eingesetzt, das ein "Prodrug" ist und im Organismus zu EE verstoffwechselt wird. EE ist bei oraler Applikation (Mensch) viel besser bioverfügbar als die o. g. natürlichen Estrogene, allerdings variiert die orale Bioverfügbarkeit individuell außerordentlich stark. Verschiedene Autoren haben auf diesen Umstand und das z. T. regellose Verhalten der Blutspiegelverläufe nach oraler Applikation dieser Substanz hingewiesen (Goldzieher J W: "Pharmacology of contraceptive steroids: A brief review" Am. f. Obstet. Gynaecol. **160**, 1260 - 1264 (1989); Goldzieher J W: "Selected aspects of the pharmacokinetics and metabolism of ethinyl estrogens and their clinical implications" Am. J. Obstet. Gynaecol. **163,** 318 - 322 (1990); Hümpel M, Täuber U, Kuhnz W, Pfeffer M, Brill K, Heithekker R, Louton T, Steinberg B: "Comparison of Serum Ethinyl Estradiol, Sex-Hormone-Binding Globulin, Corticoid-Binding Globulin and Cortisol Levels in Women Using Two Low-Dose Combined Oral Contraceptives" Horm. Res. **33**, 35 - 39 (1990); Kuhnz W, Louton T, Back D J, Michaelis K: "Radioimmunological Analysis of Ethinylestradiol in Human Serum" Arzneim.-Forsch./Drug Res. **43 (1)**, Nr. 1, 16 - 21 (1993) ).

Bei oraler Anwendung gelangen Wirkstoffe nach Resorption aus dem Darmlumen über die Leber in den Organismus. Für östrogene Wirkstoffe ist diese Tatsache von besonderer Bedeutung, da die Leber ein Erfolgsorgan für Estrogene ist und deren orale Gabe und die damit verbundene Leberpassage zu starken Estrogeneffekten in der Leber führt.

Zu den Sekretionsaktivitäten der menschlichen Leber die durch Estrogene reguliert werden, gehören u. a. die Synthesen der Transportproteine CBG, SHBG, TBG, das Angiotensinogen, verschiedene Faktoren, die in der Physiologie der Blutgerinnung eine wichtige Rolle spielen und die Lipoproteine.

Werden dem weiblichen Organismus natürliche Estrogene unter Umgehung der Leberpassage zugeführt, z. B. durch transdermale Applikation, so bleiben die genannten Leberfunktionen praktisch unverändert. Therapeutisch aequivalente Dosen natürlicher Estrogene führen bei oraler Applikation zu deutlichen Reaktionen hepatischer Parameter: Anstieg von SHBG, CBG, Angiotensinogen, HDL (high density lipoproteins). Deutlich stärker ausgeprägt als bei natürlichen Estrogenen sind entsprechende hepatische Estrogeneffekte bei equinen Estrogenmischungen, sog. konjugierte Estrogene (Campbell S, Whitehead M I: "Potency and hepato-cellular effects of estrogens after oral, percutaneous, and subcutaneous administration" International Congress on the Menopause (3rd: Ostend, Belgium, 1981), Workshop 12, 103 - 125 in The controversial climateric / MTP Press Lancaster 1982, editors Van Keep P A, Utian W H, Vermeulen). Noch stärkere hepatische Estrogenität besitzen das Ethinyl-Estradiol und das DES. Bezogen auf antigonadotrope Eigenschaften ist das EE in der Leber ca. 4-18 mal stärker estrogen wirksam als oral verabreichte natürliche Estrogene (Campbell, S. et al. ibid.). Es liegt also eine sehr ungünstige Dissoziation von Eigenschaften vor, da die erwünschten systemischen Effekten (Effekte in Genitaltrakt, Knochen, zentralem Nervensystem) gegenüber den unerwünschten hepatischen zurücktreten.

In der HRT und für die Kontrazeption werden Estrogene ganz überwiegend in Kombination mit einem Gestagen eingesetzt, z. B. Levonorgestrel, Desogestrel, Norethisteron, Medroxyprogesteronazetat, Megestrol, Cyproteronazetat., Chlormadinonazetat, Dienogest, Drospirenone. Im Falle einer kontrazeptiven Strategie wird durch die Kombination von Estrogen und Gestagen ein Synergismus bei der Unterdrückung der Ovulation erreicht. Ein zweiter wichtiger Aspekt der Kombination von Estrogen und Gestagen ist die Umwandlung der Uterusschleimhaut in Analogie zu den Vorgängen, die physiologischerweise in der Lutealphase des normalen Zyklus ablaufen.

Die Interaktion beider Hormontypen verhindert ein Überschießen von Estrogeneffekten in diesem Gewebe, die erwiesenermaßen die Entstehung von Endometriumskarzinomen begünstigen. Zudem wird das Endometrium in einen Zustand versetzt, der nach dem Absetzen der Behandlung zu einer "menstruellen" Blutung führt.

In der HRT ist der entscheidende Aspekt der Kombination mit einem Gestagen die Hemmung der Proliferationswirkung im Endometrium. Die sonstigen Interaktionen dieser Kombination sind für die Erreichung der therapeutischen Ziele ohne Belang oder sogar problematisch. Es ist in der medizinischen Wissenschaft nicht umstritten, daß bei disponierten Frauen durch die Kombination mit einem Gestagen der positive Effekt einer Estrogengehandlung stark beeinträchtigt werden kann (Breckwoldt et al.: "Consensus der Menopause Gesellschaft deutschsprachiger Länder" in Menopause 6 / Aesopus Verlag GmbH Basel 173 - 177 (1993), Editor Lauritzen C). Das Auftreten depressiver Verstimmungen ist hierfür ein Beispiel. Mögliche negative Effekte der Kombination im Vergleich zu einer reinen Estrogentherapie sind Gegenstand anhaltender Kontroversen im Hinblick auf kardiovaskuläre Morbidität und Mortalität (Lobo R A, Whitehead M: "Too much of a good thing? Use of progestogens in the menopause: an international consensus statement" Fertility and Sterility **51**, No. 2, Feb. 1989; Kuhl H: "Hormonale Kontrazeption und Substitutionstherapie: Die Bedeutung des Gestagens für kardiovaskuläre Erkrankungen" Geburts. u. Frauenheilk. **52**, 653 - 662 (1992)). Ein weiteres Problemfeld ist der Effekt einer Kombination von Estrogen und Gestagen auf die Promotion latent vorhandener Mammakarzinome. In der Milchdrüse spielt Progesteron eine Rolle beim Aufbau der Drüse in der Gravidität. Entsprechend wird seine Rolle als Mitoseauslösender Faktor in diesem Organ zum Teil ähnlich betrachtet wie die Rolle der Estrogene im Uterus (Zumoff B: "Biological and endocrinological insights into the possible breast cancer risk from menopausal estrogen replacement therapy" Steroids **58**, 196 - 204 (1993); Said T K, Conneely O M, Medina D, O'Malley B W, Lydon J P: "Progesterone, in Addition to Estrogen, Induces Cyclin D1 Expression in the Murine Mammary Epithelial Cell, *in Vivo* Endocrinology **138,** No. 9, p 3933 (1997); von Schoultz B, Söderqvist G, Tani E, Skoog L: "Effects of female sex steroids on breast tissue" European Journ. of Obstet. & Gynaecol. and Reproductive Biol. **49,** p 55 (1993)).

Um erwiesenen Nachteilen und genannten Unsicherheiten einer Gestagenanwendung in der HRT auszuweichen sind neue Behandlungsstrategien erforderlich, die auf möglichst niedrige oder lokale Gestagenbehandlung abzielen, wobei an der prinzipiellen Notwendigkeit des Gestagenzusatzes kein Zweifel bestehen kann.

Die pharmakokinetischen und pharmakodynamischen Schwachstellen natürlicher und synthetischer Estrogene haben auch eine große klinische Bedeutung. Im Falle der Estrogentherapie mit hoch dosierten Estrogenen sind thromboembolische Erkrankungen mit tödlichem Ausgang eine bekannte Komplikation. In abgeschwächter Form bestimmt dieses Nebenwirkungspotential von herkömmlichen Estrogenen die Strategie der oralen hormonalen Kontrazeption. Im Hinblick auf erwünschte kontrazeptive Effekte, die Erhaltung des monatlichen Menstruationsgeschehens ist die Beachtung des Potentials von Nebenwirkungen eine Gratwanderung.

Die Therapie mit natürlichen Estrogenen erfordert mit heutigen Technologien durchweg individuelle Dosisanpassungen. Entsprechende Behandlungen sind mit großen Unsicherheiten behaftet und beinhalten konkret die Gefahr von Über- und Unterdosierung. Die orale Therapie ist auch bei Anwendung natürlicher Estrogene (Estradiol, Estradiolvalerat, Estronsulfat, sogenannte konjugierte Estrogene) nachweislich mit unerwünschten hepatischen Effekten belastet. Es ist zudem davon auszugehen, daß sich die unphysiologischen starken Schwankungen der Blutspiegel der applizierten Estrogene und ihrer aktiven Metabolite negativ für das Erreichen der therapeutischen Ziele auswirken. Konkret heißt das, daß die konventionelle orale HRT hinter ihren theoretischen Möglichkeiten zurückbleibt.

Die transdermale HRT oder andere parenterale Techniken (Implantate, Injektionen) der Hormonapplikation vermeiden einige der für die orale HRT diskutierten Nachteile. Sie haben aber den Nachteil, daß sie nur mit Hilfe eines Arztes anwendbar sind (Injektionen, Implantate) oder in ihrer Anwendung eine erhöhte Belastung darstellen, die zum Verlassen der Therapie führen, so dass der Nutzen der HRT für Gesundheit und Lebensqualität verloren geht.

Aus der WO-A 9501161 ist eine Packung zur Verwendung bei der Hormonsubstitutionstherapie bekannt, bei der Estrogene, insbesondere Estradiol in Form eines subdermalen Implantats verabreicht wird, zusammen mit einem Progestin, welches mittels eines intrauterinen Freisetzungssystems verabreicht wird. Eine derartige Packung hat den Nachteil, dass jedenfalls das Implantat durch einen Arzt eingesetzt werden muß.

Ein weiterer Versuch, die Nachteile der Hormonsubstitutionstherapie zu überwinden, besteht gemäß der WO 97/33589 in der Verwendung von biogenen Estrogensulfamaten. Deren positive Eigenschaften werden in *Expert Opinion Invest. Drugs (1989),* 7(4), pp. 575-589 und *Journal of Steroid Biochemistry and Molecular Biology (1995), 55 (3-4), pp. 395*-403, beschrieben, wobei Versuche an Ratten zugrunde gelegt wurden. Nachteilig ist gemäß den Versuchsergebnissen jedoch, dass eine tägliche oder sogar mehrmals tägliche orale Gabe notwendig erscheint.

Aufgabe der vorliegenden Erfindung ist es, die Nachteile bei der bekannten Hormonsubstitutionstherapie (HRT) zu überwinden.

Die Aufgabe wird erfindungsgemäß durch die Verwendung von biogenen Estrogensulfamaten zur Herstellung von Arzneimitteln zur oralen, diskontinuierlichen Applikation zur Hormonsubstitutionstherapie gelöst, wobei die einzelnen Applikationen einen Abstand von 2 bis 40 Tagen aufweisen.

Es ist erfindungsgemäß bevorzugt, dass das biogene Estrogensulfamat Estronsulfamat, Estradiolsulfamat, Estriolsulfamat oder ein N-Acylsulfamat von Estron, Estradiol oder Estriol mit bis zu 7 C-Atomen in der Acylkette oder eine Kombination aus zwei oder mehreren der genannten Wirkstoffe ist. Besonders bevorzugt sind N-Acetyl- und N-Propionylderivate der Sulfamate.

Vorteilhafterweise wird bei der erfindungsgemäßen Verwendung der Estrogensulfamte bzw. deren N-Acylderivate zusätzlich mindestens ein Gestagen appliziert.

Erfindungsgemäß bevorzugt sind hierbei als Gestagene Levonorgestrel, Desogestrel, Norethisteron, Medroxyprogesteronazetat, Megestrol, Cyproteronazetat, Chlormadinonazetat, Dienogest, Drospirenon oder eine Kombination aus zwei oder mehreren der genannten Wirkstoffe.

Dabei ist es erfindungsgemäß besonders bevorzugt, daß man das Gestagen kontinuierlich in Form eines Implantats oder in Form eines intrauterinen Freisetzungssystems (IUD) oder in Kombination der genannten Applikationsarten appliziert.

Bei ovariektomierten Ratten wurde nach Behandlung mit Estradiolsulfamat eine starke orale östrogene Wirkung beobachtet. Im Vergleich Estradiol in äquimolaren Dosis wurden nach Estradiolsulfamat (J995) höhere und länger anhaltende Blutspiegel von Estradiol und Estron festgestellt. Diese Freisetzungsvorgänge waren nach 24 Stunden beendet. Auch sehr hohe Dosierungen von J995 führten nicht zu einer Verlängerung von Estrogenwirkungen.

Überraschend wurde nun gefunden, daß die Freisetzung der genannten Hormone beim Menschen aus dem Sulfamatprodrug viel langsamer verläuft als bei der Ratte.

Überraschend konnte die Dauer von Estrogenfreisetzung und Hormonwirkung durch die Höhe der Dosis beeinflußt werden, ohne daß exzessive Wirkstoffspiegel oder Effekte auftraten.

Auch 4 Wochen nach einmaliger Applikation wurden pharmokodynamisch relevante Blutspiegel gemessen.

Bei täglicher Behandlung mit niedrigen Dosierungen (100 µg J995/Tag) konnten völlig gleichmäßige Wirkstoffspiegel (Estradiol, Estron) aufgebaut und deren biologische Relevanz nachgewiesen werden.

Überraschenderweise wurde gefunden, daß bei vergleichbaren Estron- und Estradiolspiegeln im Blut von Frauen nach oraler Behandlung mit Estradiolvalerat beziehungsweise Estradiolsulfamat letzteres circa Faktor 10 niedrigere Spiegel von Estronsulfat induziert. Da dieser Estrogenmetabolit im Verdacht steht das Wachstum latent vorhandener Mammakarzinome zu promovieren, ist die Beobachtung niedriger Spiegel von Estronsulfat ein überraschener Vorteil gegenüber der konventionellen oralen HRT. Hinsichtlich Estronsulfaterhöhung verhalten sich Estradiolsulfamat und transdermale Therapie vergleichbar. Dies ist als erhebliche Verbesserung oraler Therapiemöglichkeiten anzusehen.

Die vorliegende Erfindung weist gegenüber dem Stand der Technik eine Reihe von Vorteilen auf. Die vorliegende Erfindung verbessert konventionelle Strategien der HRT unter allen diskutierten Problembereichen konventioneller HRT.

Die Compliance wird erhöht. Es wird erfindungsgemäß eine HRT nachgewiesen, welche die Anwendungsfreundlichkeit der oralen HRT erhält oder sogar verbessert durch die Option einer diskontinuierlichen Therapie, zum Beispiel durch wöchentliche oder monatliche Einnahmeintervalle als Alternative zu täglicher Behandlung.

Auch wird durch die erfindungsgemäße Verwendung die Pharmakodynamik erheblich verbessert. Die Freisetzung von Estradiol oder Estron aus Sulfamatprodrug bewirkt, daß hepatische Estrogenwirkung bei therapeutischen Dosen nicht zu erwarten ist. Dies ist ein wesentlicher Fortschritt im Vergleich zur konventionellen oralen HRT.

Ferner bleiben die Spiegel an Estronsulfat weit unter denen einer konventionellen oralen HRT. Estronsulfat wird von (latenten) Mammakarzinom durch deren hohe Sulfataseaktivität gespalten. Es besteht die Gefahr einer Promotionswirkung durch konventionelle orale HRT. Diese wird durch die vorliegende Erfindung reduziert.

Auch in Bezug auf die Pharmakokinetik weist die erfindungsgemäße Verwendung erhebliche Vorteile auf. Durch erfindungsgemäße langsame Freisetzung aus dem Sulfamatprodrug beim Menschen können sehr gleichmäßige, exakt definierte Spiegel natürlicher Estrogene im Blut aufgebaut werden.

Langsame Freisetzung natürlicher Estrogenen, in Verbindung mit einer erfindungsgemäß hohen oralen Bioverfügbarkeit des Steroidanteils des applizierten Estradiolsulfamates, erlaubt die Anwendung in größeren Intervallen.

Durch die Höhe der Dosis kann erfindungsgemäß die Dauer der Hormonwirkung gesteuert werden. Sehr niedrige Dosierungen (20-300 µg) sind optimal für 1-3-tägige Behandlungsintervalle, mittlere Dosierungen (0,5-5,0 mg/Tag) eignen sich erfindungsgemäß für 5-10-tägige Behandlungsintervalle, höhere Dosierungen (2,0-20 mg/Tag) sind erfindungsgemäß für Behandlungsintervalle von 20-40 Tagen geeignet

Auch wird die Compliance bei einer zusätzlichen Gestagenbehandlung erheblich verbessert. Die erfindungsgemäße HRT wird in ihrer Akzeptanz verbessert durch Gestagenbehandlung mittels IUS oder Implantat, die gestagene Wirkstoffe nach einmaliger Einbringung durch den Arzt kontinuierlich über längere Zeit freisetzen. So bleibt erfindungsgemäß der Vorteil der Bequemlichkeit einer diskontinuierlichen Estrogenbehandlung erhalten. In Kombination mit konventioneller Estrogentherapie läßt sich ein entsprechender Vorteil nicht realisieren

Auch wird durch die erfindungsgemäße Gestagenbehandlung die Pharmakodynamik verbessert. Durch niedrige systemische Substanzfreisetzung (Implantat) oder die Beschränkung der Wirkstoffreisetzung auf den Uterus wird die günstige Estrogenwirkung für die HRT nicht beeinträchtigt, Probleme, die sich aus systemischen Gestageneffekten ergeben könnten werden auf ein Minimum reduziert

Die Sulfamate der biogenen Estrogene und deren N-Acyl-Derivate sind an sich bekannt. Die Herstellung dieser Verbindungen erfolgt in an sich bekannter Weise auf synthetischem Wege aus den biogenen Estrogenen. Dabei werden gegebenenfalls ein Teil der freien OH-Gruppen oder andere reaktive Gruppen mit geeigneten Schutzgruppen versehen, welche nach erfolgter Synthese wider abgespalten werden. Zur Darstellung der N-Acylderivate der Sulfamate werden die entsprechend N-acylierten Amidoschwefelsäurederivate zur Synthese eingesetzt.

Die Herstellung der für die erfindungsgemäße Verwendung erforderlichen pharmazeutischen Zusammensetzungen ist dem Fachmann an sich bekannt. Sie entspricht denen, wie sie beispielsweise für die Herstellung von Oralia für die hormonale Kontrazeption bekannt sind.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Entsprechende Tabletten können beispielsweise durch Mischen des Wirkstoffs mit bekannten Hilfsstoffen, beispielsweise inerten Verdünnungsmitteln wie Dextrose, Zukker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Die Herstellung von Implantaten oder intrauterinen Freisetzungssystemen (IUD) für die Applikation des Gestagens sind dem Fachmann gleichfalls bekannt.

### Die nachfolgenden Beispiele erläutern die Erfindung:

In den folgenden Beispielen wurde die Bestimmung der Plasmaspiegel der Estrogene mit an sich bekannten und anerkannten, validierten Methoden durchgeführt.

### Beispiel 1

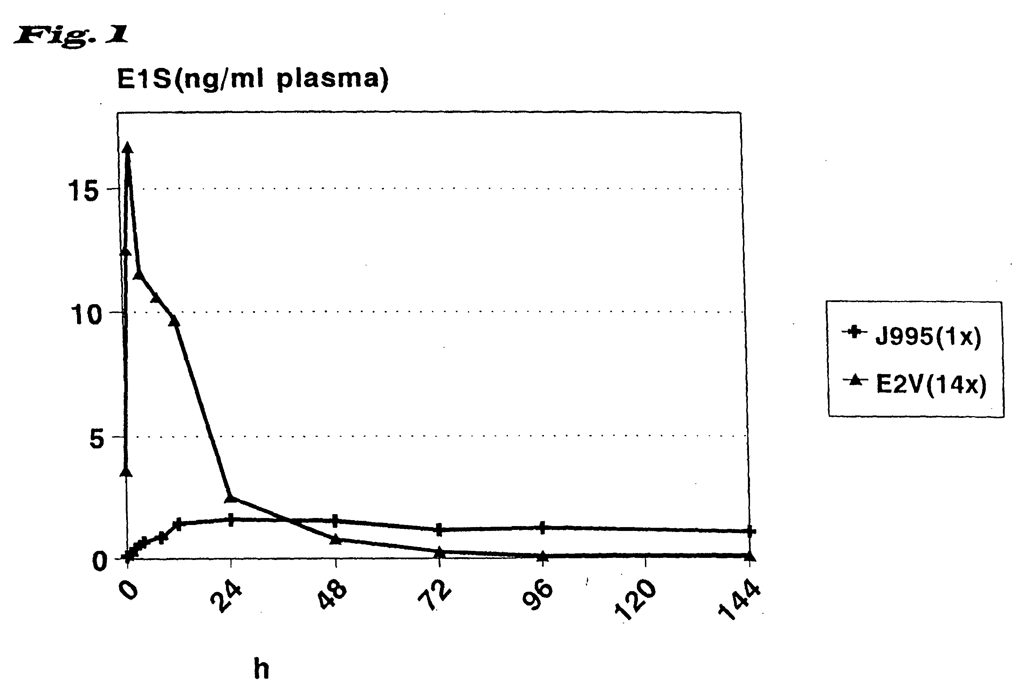

### Vergleich Estradiolvalerat und Estradiolsulfamat bezüglich induzierter Estronsulfatspiegel bei postmenopausalen Frauen (Fig. 1)

Nach einmaliger oraler Applikation von 2 mg Estradiolvalerat (EV)beziehungsweise 2 mg Estradiolsulfamat steigen die Estronsulfatspiegel im Plasma an. Dieser Anstieg fällt bei EV fiel stärker aus, ist aber auch rascher beendet, so daß 48 Stunden nach Applikation die Estronsulfatspiegel nach Estradiolsulfamat höher liegen als nach EV.

### Beispiel 2

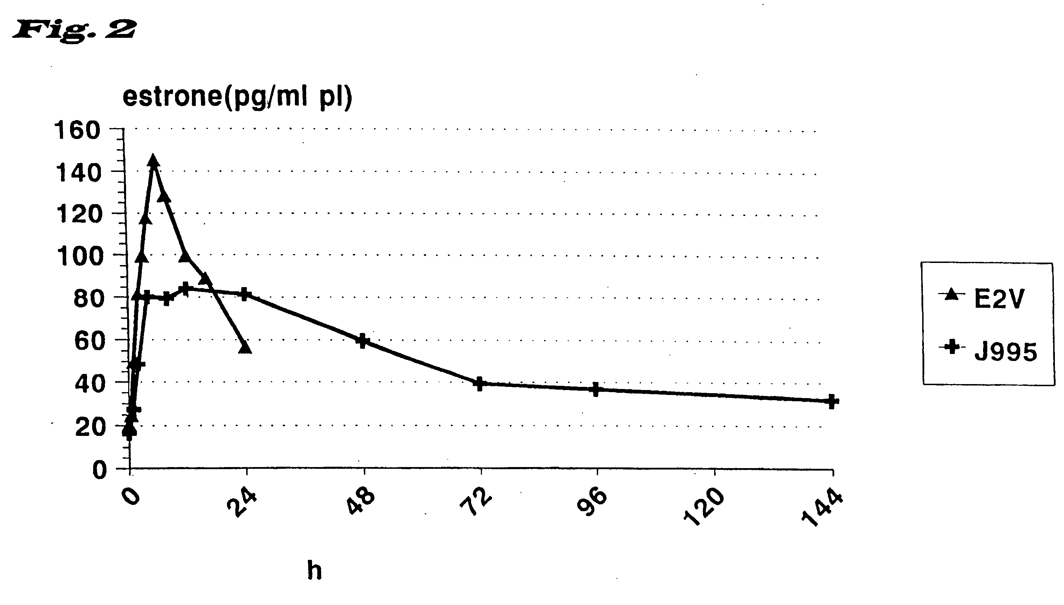

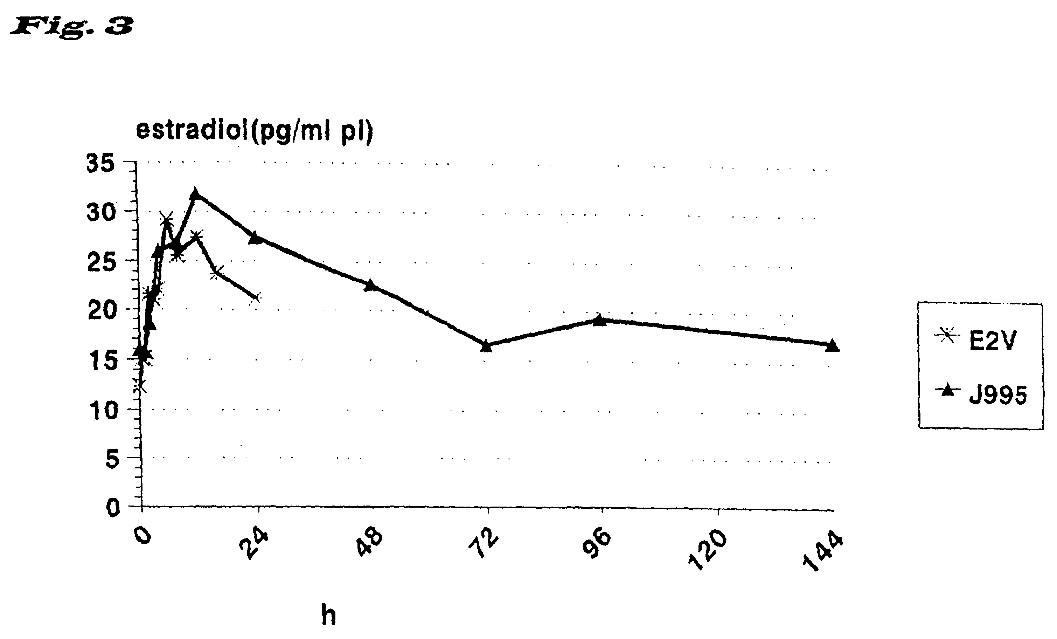

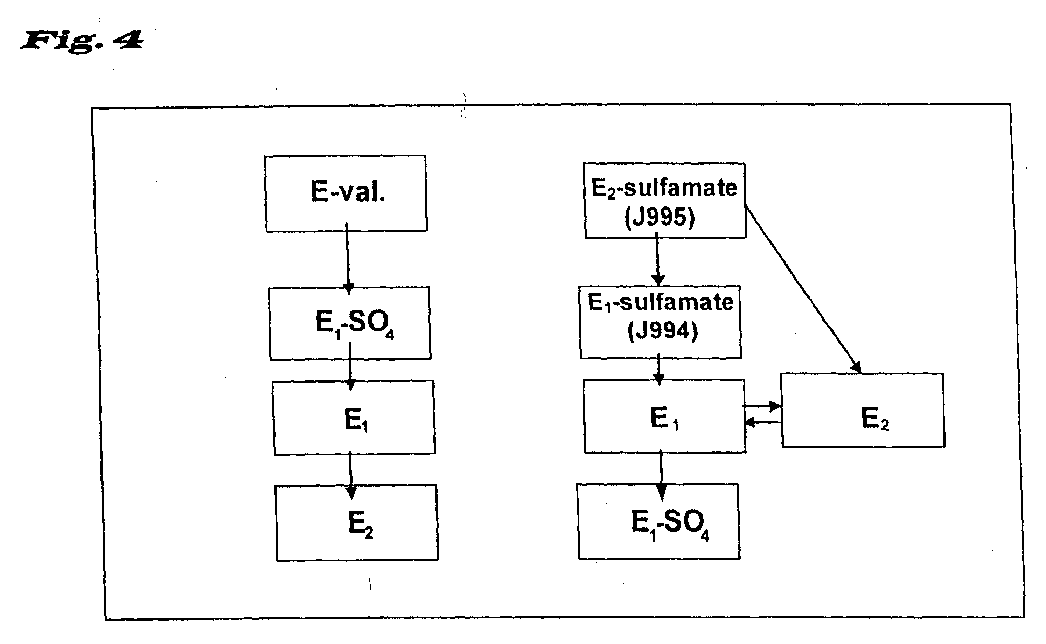

### Vergleich Estradiolvalerat und Estradiolsulfamat bezüglich induzierter Estronspiegel bei postmenopausalen Frauen (Fig. 2)

Nach einmaliger oraler Applikation von 2 mg Estradiolvalerat (EV)beziehungsweise 2 mg Estradiolsulfamat steigen die Estronspiegel im Plasma an. Dieser Anstieg fällt bei EV initial stärker aus, ist aber auch rascher beendet. Bereits 24 Stunden nach Applikation liegen die Estronspiegel nach Estradiolsulfamat höher liegen als nach EV. Diese Erhöhung hält lange an.

### Beispiel 3

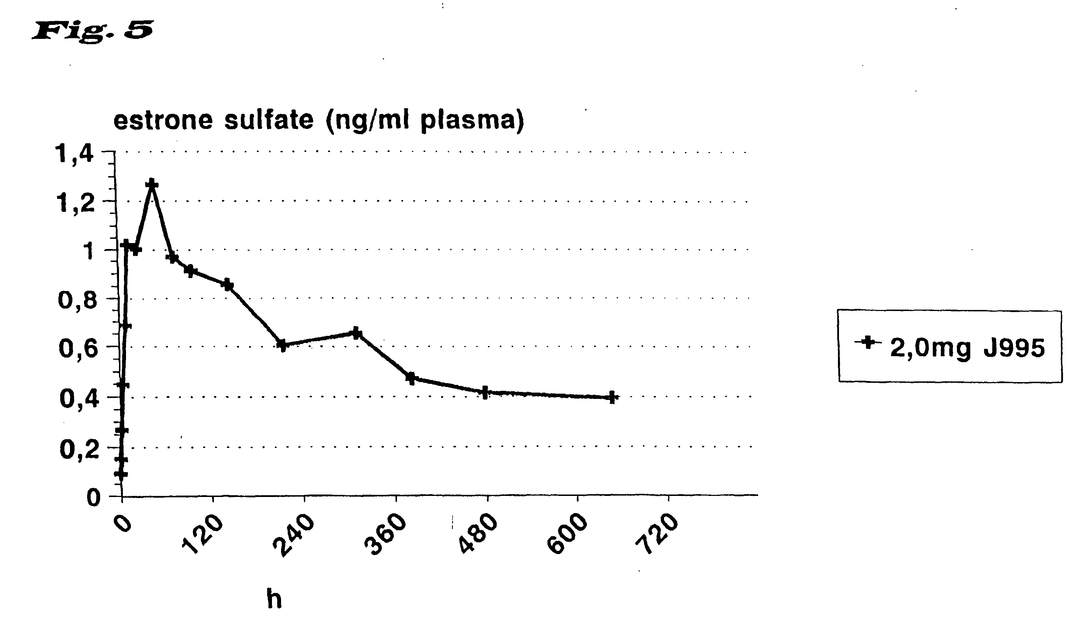

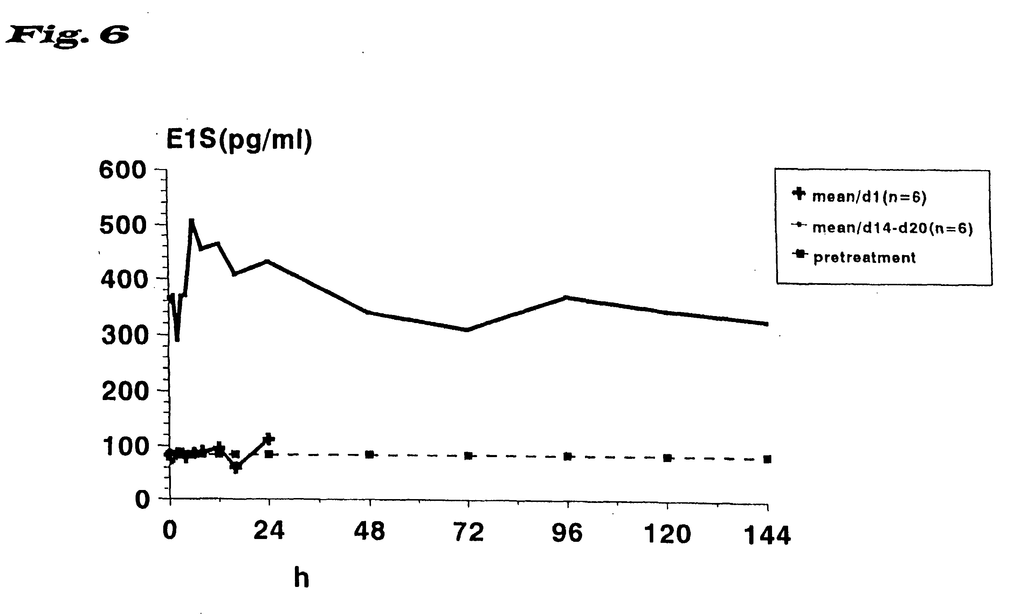

### Vergleich Estradiolvalerat und Estradiolsulfamat bezüglich induzierter Estradiolspiegel bei postmenopausalen Frauen (Fig. 3)

Nach einmaliger oraler Applikation von 2 mg Estradiolvalerat (EV)beziehungsweise 2 mg Estradiolsulfamat steigen die Estradiolspiegel im Plasma an. Dieser Anstieg fällt bei EV schwächer aus, ist auch viel rascher beendet. Auch eine Woche nach der Behandlung bestehen Estradiolblutspiegel, die deutlich über den Ausgangswerten liegen.

### Beispiel 4

### Mechanismus der Generierung von Estron und Estradiol nach oraler Anwendung von Estradiolvalerat und Estradiolsulfamat (Fig. 4)

Durch EV wird im Blut ein "pool" von Estronsulfat erzeugt. Aus diesem wird durch Hydrolyse Estron freigesetzt, das wiederum zu einem kleinen Anteil zu Estradiol verstoffwechselt wird. Die im Vergleich zu EV sehr niedrigen Spiegel von Estronsulfat nach Estradiolsulfamat, aber die insgesamt höhere Freisetzung (Fläche unter der Kurve) von Estron nach Estradiolsulfamat belegt die direkte Umwandlung des Sulfamates zu therapeutisch relevanten Estrogenen Estron und Estradiol. Estronsulfat ist in diesem Fall nur Hauptmetabolit der aus dem Sulfamat entstandenen Estrogene.

### Beispiel 5

### Dauer der Estrogenfreisetzung aus Estradiolsulfamat reflektiert durch erhöhte Estronsulfatspiegel bei postmenopausalen Frauen (Fig. 5)

Die mittleren Estronsulfatspiegel von drei postmenopausalen Frauen weisen nach einmaliger Applikation von 2 mg über 600 Stunden eine deutliche Erhöhung gegenüber den Ausgangswerten auf.

## Patentansprüche

1. Verwendung von biogenen Estrogensulfamaten zur Herstellung von Arzneimitteln zur oralen, diskontinuierlichen Applikation zur Homonsulostitutionstherapie, wobei die einzelnen Applikationen einen Abstand von 2 bis 40 Tagen aufweisen.

2. Verwendung von biogenen Estrogensulfamaten nach Anspruch 1, **dadurch gekennzeichnet, daß** das biogene Estrogensulfamat Estronsulfamat, Estradiolsulfamat, Estriolsulfamat oder ein N-Acylsulfamat von Estron, Estradiol oder Estriol mit bis zu 7 C-Atomen in der Acylkette oder eine Kombination aus zwei oder mehreren der genannten Wirkstoffe ist.

3. Verwendung nach einem der voranstehenden Ansprüche, **dadurch gekennzeichnet, daß** man zusätzlich mindestens ein Gestagen appliziert.

4. Verwendung nach Anspruch 3, **dadurch gekennzeichnet, daß** das Gestagen Levonorgestrel, Desogestrel, Norethisteron, Medroxyprogesteronazetat, Megestrol, Cyproteronazetat, Chlormadinonazetat, Dienogest, Drospirenon oder eine Kombination aus zwei oder mehreren der genannten Wirkstoffe ist.

5. Verwendung nach Anspruch 4, **dadurch gekennzeichnet, daß** man das Gestagen kontinuierlich in Form eines Implantats oder in Form eines intrauterinen Freisetzungssystems (IUD) oder in Kombination der genannten Applikationsarten appliziert.

6. Verwendung nach einem der Ansprüche 1 bis 5, **dadurch gekennzeichnet, daß** der Applikationsabstand 5 bis 10 Tage und die für den Applikationsabstand 0,5 - 5,0 mg biogenes Estrogensulfamat beträgt.

7. Verwendung nach einem der Ansprüche 1 bis 5, **dadurch gekennzeichnet, daß** der Applikationsabstand 20 bis 40 Tage und die Eimmaldosis für den Applikationsabstand 2,0 - 20,0 mg biogenes Estrogensulfamat beträgt.

## Claims

1. Use of biogenic estrogen sulfamates for the production of drugs for oral, discontinuous administration in hormone replacement therapy, wherein single administrations are effected at intervals of from 2 to 40 days.

2. The use of biogenic estrogen sulfamates according to claim 1, **characterized in that** the biogenic estrogen sulfamate is estrone sulfamate, estradiol sulfamate, estriol sulfamate, or an N-acylsulfamate of estrone, estradiol or estriol with up to 7 C atoms in the acyl chain, or a combination of two or more of the above-mentioned active substances.

3. The use according to any of the preceding claims, **characterized in that** at least one gestagen is administered in addition.

4. The use according to claim 3, **characterized in that** the gestagen is levonorgestrel, desogestrel, norethisterone, medroxyprogesterone acetate, megestrol, cyproterone acetate, chlormadinone acetate, dienogest, drospirenone, or a combination of two or more of the above-mentioned active substances.

5. The use according to claim 4, **characterized in that** the gestagen is administered continuously in the form of an implant or in the form of an intrauterine release system (IUD) or in a combination of the above-mentioned types of administration.

6. The use according to any of claims 1 to 5, **characterized in that** the administration interval is 5 to 10 days and the single dose for said administration interval is 0.5 to 5.0 mg of biogenic estrogen sulfamate.

7. The use according to any of claims 1 to 5, **characterized in that** the administration interval is 20 to 40 days and the single dose for said administration interval is 2.0 to 20.0 mg of biogenic estrogen sulfamate.

## Revendications

1. Utilisation de sulfamates d'oestrogène biogènes pour préparer des médicaments destinés à une administration orale discontinue pour un traitement hormonal de substitution, moyennant quoi les différentes administrations s'effectuent à un intervalle de 2 à 40 jours.

2. Utilisation de sulfamates d'oestrogène biogènes selon la revendication 1, **caractérisée en ce que** le sulfamate d'oestrogène biogène est du sulfamate d'oestrone, du sulfamate d'oestradiol, du sulfamate d'oestriol ou un N-acylsulfamate d'oestrone, d'oestradiol ou d'oestriol comportant jusqu'à 7 atomes de C dans la chaîne acyle ou une combinaison de deux ou plusieurs des substances mentionnées.

3. Utilisation selon une des revendications précédentes, **caractérisée en ce qu'**on administre en plus au moins un gestagène.

4. Utilisation selon la revendication 3, **caractérisée en ce que** le gestagène est du lévonorgestrel, du désogestrel, du noréthistérone, de l'acétate de médroxyprogestérone, du mégestrol, de l'acétate de cyprotérone, de l'acétate de chlormadinone, du diénogest, du drospirénone ou une combinaison de deux ou plusieurs des substances mentionnées.

5. Utilisation selon la revendication 4, **caractérisée en ce que** le gestagène est administré en continu sous forme d'un implant ou sous forme d'un système de libération intra-utérin (IUD) ou en combinant les deux types d'administration.

6. Utilisation selon une des revendications 1 à 5, **caractérisée en ce que** l'intervalle d'administration est de 5 à 10 jours et la dose utilisée à chaque fois dans ledit intervalle d'administration est de 0,5 à 5,0 mg de sulfamate d'oestrogène biogène.

7. Utilisation selon une des revendications 1 à 5, **caractérisée en ce que** l'intervalle d'administration est de 20 à 40 jours et la dose utilisée à chaque fois dans ledit intervalle d'administration est de 2,0 à 20,0 mg de sulfamate d'oestrogène biogène.
